# EUROPEAN PATENT APPLICATION

(11) **EP 1 400 235 A1**
(43) Date of publication of application: **24.03.2004**
(21) Application number: 01943557.7
(22) Date of filing: 26.06.2001
(51) Int. Cl.: A61K 7/06

(54) **HAIR PRODUCT MADE FROM AQUEOUS PLANT EXTRACTS**

(30) Priority: 16.05.2001 ES 200101108
(71) Applicant: Garcia Lozano, Marina, 02005 Albacete (ES)
(72) Inventor: Garcia Lozano, Marina, 02005 Albacete (ES)
(74) Representative: Carpintero Lopez, Francisco
(86) International application number: PCT/ES2001/000257
(87) International publication number: WO 2002/092030

(57) **Abstract**

The hair product consists of a mixture of aqueous plants produced by boiling a ground mixture of plants of birch, burdock, horsetail, lavender, marjoram, nettle, cat's-tail, rosemary and thyme. This hair product is suitable for hair care and treatment.

## Description

### AREA OF THE INVENTION

This invention refers to a hair product comprised of a blend of aqueous extracts produced by boiling together a mixture of ground plants of birch, burdock, horsetail, lavender, marjoram, nettle, cat's-tail, rosemary and thyme. This product is suitable for the treatment and care of hair.

### BACKGROUND OF THE INVENTION

Some illnesses, including skin diseases and internal illnesses, situations of stress and/or unfavourable environments, together with the use of inappropriate hair products that, owing to their poor quality, incorrect composition and/or infrequent use, create problems for the user. These problems include damaged hair, dandruff, greasy hair and scalp and alopecia (hair loss).
Numerous compositions are known to condition and restructure damaged hair ["Cosmetología Teórico-Práctica", 3^{a} Edicion, 1985, Publicaciones del Consejo General de Colegios Oficiales de Farmacéuticos, pages 252-260], and several anti-dandruff preparations ["Cosmetología Teórico - Práctica", cited *supra,* pages 260-264], and compositions to treat greasy hair and scalp ["Cosmetología Teórico - Práctica", cited *supra,* pages 264-270].

Suitable compositions are also available to reduce or prevent hair loss based on pantotenic acid and its salts. Minioxydyl (2,4-diamino-6-piperidine-pirimidine-3-oxide) is a product used in preparations to stimulate hair emergence.

Also, a number of hair lotions are known based on medicinal plants that reduce or prevent hair loss, that contain extracts of yarrow (*Achillea millefolium L*.) and artemisia (*Artemisa abrotonum L.*), and hair lotions that stimulate hair emergence based on basil (*Ocimum basilicum L*.), boxwood (*Buxus sempervivens L.*) and Centaury menor (*Erythraea centaurium Pres.*) ["Secretos y Virtudes de las Plantas Medicinales", selected passages from the Reader's Digest, 1982, page 383].

The Spanish patent application P9802211 describes a natural hair product suitable for hair treatment and care that consists of a blend of aqueous extracts made by boiling together a mixture of plants comprised of birch, burdock, horsetail, lavender, marjoram, nettle, cat's-tail, rosemary and thyme.

Although several products have been developed for the treatment and care of hair, there is still the need to look for other products to increase the range of treatments available for this purpose.

It has now been found, surprisingly, that the addition of two plants (horsetail and cat's-tail) to the mixture of plants described in Spanish patent P9802211 gives the hair a silky appearance, helps to clean the scalp and to treat dandruff.

Therefore, the present invention provides a hair product, of natural origin, appropriate for treating and caring for hair that consists of a blend of aqueous extracts produced by boiling together a mixture of plants.

To make the hair product of the invention, the plants birch, burdock, horsetail, lavender, marjoram, nettle, cat's-tail, rosemary and thyme were used.

Birch (*Betula alba L.*) has antiseptic, cicatrizing and diuretic properties.

Burdock (*Arctium lappa L.*) is usually used as an antidiabetic, antidiarrheic and antiseptic agent.

Horsetail (*Equisetum arvense L.*) is used as a mineralizing agent, diuretic, haemostatic and cicatrizing agent.

Lavender (*Lavandula officinalis Chaix*) has antiseptic, carminative, diuretic and insecticide properties.

Marjoram (*Origanum majorana L.*) is used to treat anxiety, asthenia, tiredness and vertigo.

Nettle (*Urtica dioica L.*) is used to treat anaemia, psoriasis and urticaria.

Cat's-tail (*Sideritis angustifolia L.*) is usually used for its digestive and cicatrizing properties and to treat intestinal inflammations and stomach ulcers.

Rosemary (*Rosmarinus officinalis L.*) is used as a cholagogue, diuretic and tonic.

Thyme (*Thymus vulgaris L.*) is usually used to treat anemia, asthenia, sinusitis and whooping cough.

### DETAILLED DESCRIPTION OF THE INVENTION

The invention provides a hair product that consists in a blend of aqueous extracts produced by boiling together a mixture of ground plants containing birch, burdock, horsetail, lavender, marjoram, nettle, cat's-tail, rosemary and thyme. The hair product provided by this invention is suitable for the treatment and care of hair.

The addition of horsetail and cat's-tail to the mixture of plants containing birch, burdock, lavender, marjoram, nettle, rosemary and thyme described in Spanish patent P9802211 is clearly beneficial since horsetail seems to improve the hair's texture and shininess giving it a silky appearance; and cat's-tail stimulates pore opening in the scalp making it easier to clean and facilitating the transport of compounds contained in the hair product of the invention to the hair follicles and also has an anti-dandruff action.

The hair product of the invention can be obtained by a procedure that includes the steps of:
a) mixing ground plants of birch, burdock, horsetail, lavender, marjoram, nettle, cat's-tail, rosemary and thyme;
b) adding water to the mixture of ground plants in stage a), at a ratio of between 30 and 45g of this ground plant mixture per litre of water;
c) boiling the mixture produced in stage b), maintaining it at this temperature for at least 5 minutes;
d) cooling the mixture produced by the boiling process in step c) to room temperature; and
e) separating the liquid produced by step d) from the solid residue.
The hair product of the invention is the liquid that results from separating the liquid in step e) that contains the water-soluble extracts present in the initial plant mixture.
More specifically, the process followed to obtain the hair product of the invention begins with the collection of plants of birch, burdock, horsetail, lavender, marjoram, nettle, cat's-tail, rosemary and thyme that make up the initial plant blend. The plants can be collected at any time of year, or during the seasons most suitable for the collection of each specific plant. All the plants that constitute the initial plant blend are common in Spain or can be easily obtained from herbalist's. Any treatise on medicinal plants such as the book "Secretos y Virtudes de las Plantas Medicinales" cited *supra,* provides information about where these plants can be found, the parts of the plants that should be used and the best time of year to collect them.

The collected plants are dried, chopped and ground by conventional methods and are, then, mixed in any appropriate weight ratio. In one application, they are mixed in equal weight proportions.

To obtain the aqueous extract of the ground initial plant blend, an appropriate amount of this mixture is boiled in an appropriate amount of water. In one specific application of this invention, the ratio between the ground plants and the water used ranges from 30 to 45g, preferably 37.5 g of this plant mixture per litre of water.
The mixture of ground plants and water is boiled for an appropriate period of time to permit extraction of the active ingredients soluble in hot water present in the initial plant blend to be extracted. In one application, this blend of ground plants and water is boiled for a period of time corresponding to at least, 20 minutes.

After boiling the plant mixture in water this is left to cool to room temperature. The ground plant remains are placed in the bottom of recipient used for the cooking process and the liquid phase is separated by conventional methods used to separate solids and liquids, for example, by filtration or sieving.
The liquid phase separated off corresponds to the hair product of the invention and contains the water soluble extracts present in the initial plant blend.

The hair product of the invention is highly stable and can be kept at room temperature, maintaining its properties for a long period of time.

The hair product of the invention is suitable for the treatment of hair, especially to avoid hair loss, enhance thickening of hair and to treat seborrea. Several trials have demonstrated the efficacy of the hair product of the invention to treat different types of alopecia both of congenital origin and that produced by stress, illness etc. Similarly, the hair product of the invention delays the appearance of grey hair since it darkens, from the root, the colour of the emerging hair giving it its natural colour. The hair product of the invention presents, among others, the following properties, it:
- reduces or prevents hair loss;
- stimulates hair growth;
- thickens the hair;
- darkens the hair [maintaining its natural colour]; and
- reduces or eliminates the grease.

The hair product is applied topically to the hair in an amount suitable to massage the head and to help the hair product penetrate the scalp. Alter leaving the product to act for an appropriate period of time the hair is rinsed and, if desired, the operation can be repeated.
Initially, the hair product should be applied daily and after dilatation of the scalp, should be applied every three days. Application of the hair product of the invention should be, completely or partially, suppressed when new emerging hairs are observed. In general, in most cases of alopecia treated with the hair product of the invention, it has been observed that after 15 days of treatment, bulbs appear under the skin, followed by down, itchiness and, finally, the appearance of hair. Similarly, several studies have shown that when the individual to be treated is completely bald, the head should be shaved on appearance of the first down. In general, the duration and intensity of the treatment depends on the area to be cared for or treated and on the criteria of the health care professional prescribing the treatment.
Moreover, the hair product of the invention can be used to prepare suitable cosmetic products to treat and care for hair. The cosmetic products that can be prepared using the hair product of the invention can be available in a wide range of forms such as shampoos, gels and hair lotions. To prepare these cosmetic products, an appropriate amount of the hair product of the invention is mixed with the necessary adjuvants and/or excipients required for the chosen formulation, as described, for example, in the book, "Cosmetología de Harry", by Wilkinson and Moore, Ediciones Diaz de Santos S.A., (1990), pages 839-994. Excipients can correspond to antioxidants, antiseptic agents, aromatizers, shampoo bases, colorants, preservatives, detergents, emulsifiers, thickeners, foaming agents, solubilizers and surfactants usually used in the cosmetics industry to produce these cosmetic products. In one specific application, this cosmetic product is a shampoo that contains, as well as a hair product of the invention, a shampoo base, for example, a conventional shampoo base that can produce foam, such as a shampoo base mainly comprised of plant extracts that enhances the beneficial effects of the hair product of the invention producing a natural shampoo base on plant extracts.

The following example that describes a procedure to obtain a hair product according to the present invention, illustrates one way to achieve the object of this invention, but in no way limits the scope of the invention.

### EXAMPLE

### Preparation of a hair product

A hair product was prepared according to the process described below.

Samples of the plants birch, burdock, horsetail, lavender, marjoram, nettle, cat's-tail, rosemary and thyme are collected and dried, chopped and mixed in quantities of equal weight. Then, 150 g of this ground blend of plants is mixed with 4 litres of water and the resulting mixture is boiled for, at least, 20 minutes. After finishing the cooking process, the resulting product is left to cool at room temperature and, then, sieved to remove the ground plant remains. The liquid phase separated off corresponds to the hair product and contains the water soluble extracts present in the initial plant blend.

## Claims

1. A hair product based on aqueous plant extracts, that consists in a mixture of aqueous extracts produced by boiling together a ground plant blend containing plants of birch, burdock, horsetail, lavender, marjoram, nettle, cat's-tail, rosemary and thyme.

2. A process to make the hair product according to claim 1 that consists in the following steps:
a) mixing ground plants of birch, burdock, horsetail, lavender, marjoram, nettle, cat's-tail, rosemary and thyme;
b) adding water to the blend of ground plants from step a), in a ratio ranging from 30 to 45g of this mixture of ground plants per litre of water;
c) boiling the mixture produced in step b) keeping it at this temperature for a period of time corresponding to, at least, 5 minutes;
d) cooling the product produced by the cooking process in step c)to room temperature; and
e) separating the liquid present in the product resulting from step d) from the solid residue.

3. Process according to claim 2, in which this plant mixture of birch, burdock, horsetail, lavender, marjoram, nettle, cat's-tail, rosemary and thyme contain equal weights of these plants ground.

4. Process according to claim 2, in which the ratio of ground plant blend to water is 37.5g of this ground plant blend per litre of water.

5. Process according to claim 2, in which this mixture of ground plants and water is boiled for a period of time of, at least, 20 minutes.

6. Process according to claim 2 in which the liquid present in the resulting product from step d) is separated from the solid residue by filtration.

7. A cosmetic product for hair care and treatment that consists of a hair product based on aqueous plant extracts according to claim 1, or obtained by a process according to any of claims 2 to 6, together with one or more adjuvants and/or acceptable excipients.

8. Cosmetic product according to claim 7, choosing between shampoos, gels and hair lotions.

9. Cosmetic product according to claim 8, in the form of shampoo, that corresponds to a hair product based on aqueous plant extracts according to claim 1, or obtained by a process according to any of claims 2 to 6, together with a shampoo base mainly comprised of plant extracts.
